# EUROPEAN PATENT SPECIFICATION

(11) **EP 0 550 612 B1**
(45) Date of publication and mention of the grant of the patent: **06.03.1996**
(21) Application number: 91917624.8
(22) Date of filing: 25.09.1991
(51) Int. Cl.: C07C 231/14, C07C 231/10, C07C 233/43

(54) **NEW PROCESS FOR PREPARING FORMOTEROL AND RELATED COMPOUNDS**
NEUES VERFAHREN ZUR DARSTELLUNG VON FORMOTEROL UND VEWANDTEN VERBINDUNGEN
NOUVEAU PROCEDE DE PREPARATION DU FORMOTEROLE ET DE COMPOSES ASSOCIES

(30) Priority: 26.09.1990 SE 9003057
(43) Date of publication of application: 14.07.1993
(73) Proprietor: Astra Aktiebolag, S-151 85 Södertälje (SE)
(72) Inventor: TROFAST, Jan, William, S-222 47 Lund (SE); JAKUPOVIC, Edib, S-155 00 Nykvarn (SE); M NSSON, Lena, Katarina, S-123 43 lvsjö (SE)
(86) International application number: SE9100643
(87) International publication number: WO9205147

(56) References cited:
- DE-C- 2 366 625
- US-A- 3 933 911
- CHEMICAL ABSTRACTS, volume 112, no. 19, page 706, 7 May 1990, Columbus, Ohio, US, abstract 178373t, L. BORRAS SOLE et al, "Preparation of N-[2-hydroxy-5-[1-hydroxy-2-[[2-(4-methoxyphenyl)-1-methylethyl]amino]ethyl]phenyl]formamide, i.e. formoterol"; & ES-A-2 005 492

## Description

The present invention relates to an improved and simplified process for the preparation of formoterol and related compounds and derivatives thereof and their pharmacologically and pharmaceutically acceptable fumarate salts and solvates thereof. Formoterol is a long-acting and highly selective bronchodilator. The β₂-adrenoceptor agonists taken by inhalation are the adminstration of choice in the symptomatic therapy of obstructive airway disease.

### Background of the invention

The administration by inhalation enables the dose to be delivered directly to the airways. By this route of adminstration, it is possible to give a small dose and thereby minimize unwanted side-effects. The drawbacks of the currently available bronchodilators are their relatively short duration of action. Studies with formoterol given by inhalation have shown it has a much longer duration than any other bronchodilators on the market. By using a compound with long duration it should be possible to avoid nocturnal asthma, so often causing considerable anxiety and debility to the patients. Formoterol gives less nocturnal waking. Formoterol has been registered for oral adminstration in Japan since 1986.

Formoterol has two asymmetric carbon atoms in the molecule. It is used as the fumarate salt of one of the two possible pairs of enantiomers of 3-formamido-4-hydroxy-α-[N[1-methyl-2-(p-methoxyphenyl)ethyl]amino-methyl]benzyl alcohol. Formoterol consists of the enantiomers, which have the RR + SS configuration.

Formoterol was first described in a Japanese patent application (Yamanouchi Pharmaceutical, Japan no 13121, priority 5 february 1972, related priorities Japan no 39416 (19 april 1972), 51013 (23 May 1972) and 52925 (27 May 1972)). The corresponding patent in Germany is DE 2 305 092.

DE 2 366 625 discloses some α-aminomethylbenzyl alcohol derivatives which are intermediates for preparing end products useful as bronchodilators. Example 3 therein refers to the preparation of 3-formamido-4-hydroxy-α-(N-benzyl-N-isopropylaminomethyl)benzylalcohol.

The hitherto published synthetic routes to formoterol involve a nucleophilic substitution reaction of an amine to a bromoketone according to:

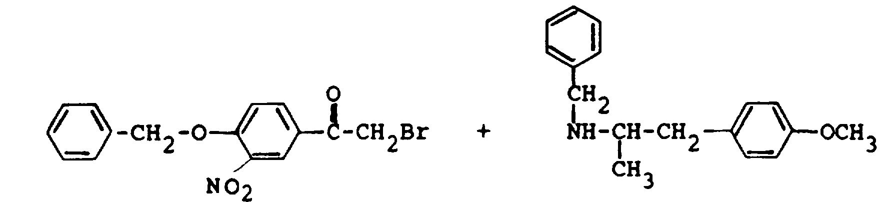

The reduction of the nitro group is accomplished by either Fe/HCl or Sn/HCl followed by formylation with a mixture of formic acid and acetic anhydride. The presence of acetic anhydride has been shown to give some undesired acetylated products. The separation of the two pairs of enantiomers is tedious and several recrystallizations are needed to obtain sufficient purity of the product.

The patent application J 50012-040 (priority date 31 May 1973) discloses a process where a substituted ketone undergoes a reductive alkylation with the appropriate amine.

ES 2 005 492 (S.A. Lasa Laboratorios) describes a process for the synthesis of formoterol characterized by a coupling reaction between 3-formamido-4-benzyloxyphenyloxirane and the unprotected 2-(4-methoxyphenyl)-1-methylethyl amine and is followed by tedious and troublesome steps (use of crown ethers, hydrofluoric acid, solvents like benzene and methylene chloride causing environmental and health problems, HCOOH/acetic anhydride etc.) to the final product.

The synthesis of formoterol and closely related compounds has also been reported in Chem. Pharm. Bull., 25(6), 1368-1377 (1977), where 3-nitro-4-benzyloxy-α-(N-substituted aminomethyl)benzyl alcohols are used as the key intermediates. Use of Raney-nickel for the reduction of the nitro group has been rejected due to difficulties in obtaining pure products without a chromatographic step. However, the general procedure for the preparation of 3-amino-4-benzyloxy-α-(N-substituted aminomethyl)benzyl alcohols reported on page 1373 of said document involves a tedious and cumbersome column chromatographic step on silica gel using benzene-ethyl acetate as the eluent.

There is a strong desire to synthesize formoterol fumarate (as dihydrate) by a simple procedure causing small environmental concerns, e.g. by elimination of solvents like benzene.

### Brief description of the invention.

The present invention is directed to a process for preparing a compound of the general formula I 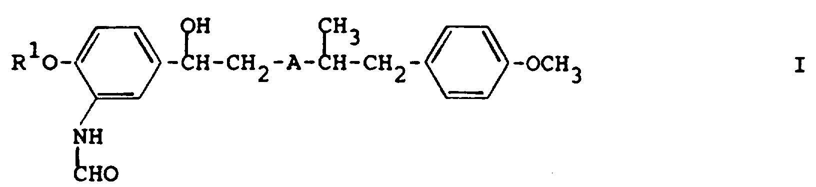 wherein R¹ is H or a straight or branched alkyl group having 1-5 carbon atoms,
A is either 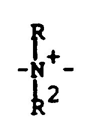 wherein R is H or a straight or branched alkyl group having 1-5 carbon atoms and R is H or a straight or branched alkyl group having 1-5 carbon atoms; or 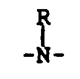 wherein R is as defined above,
or a pharmaceutically acceptable fumarate (and/or a solvate of the fumarate) thereof, in the form of the pure racemates, by
a) reacting 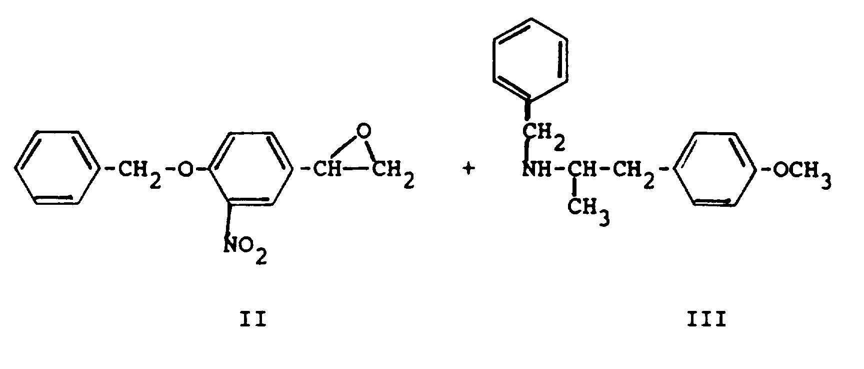 to give compound IV 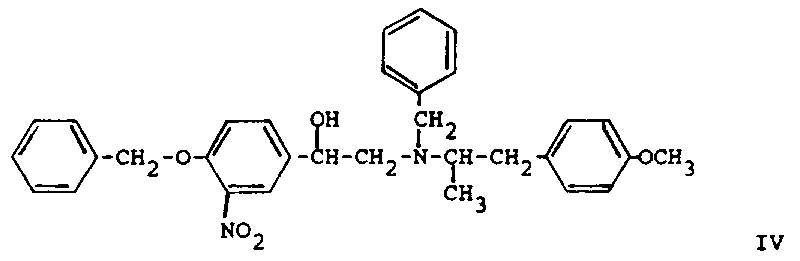
b) reducing and formylating compound IV to give compound V 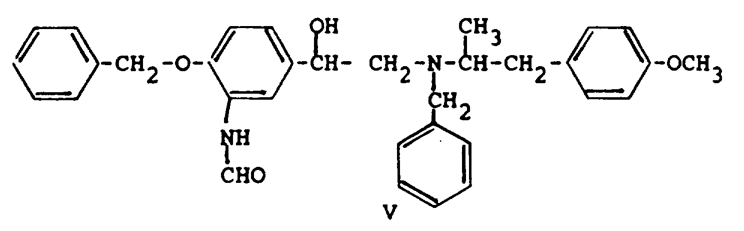
c) forming the fumarate of compound V and resolving the racemates by crystallization,
d) extracting the base of the desired racemate of compound V,
e) hydrogenolyzing of the protecting benzyl groups of said base of compound V to obtain compound I wherein A is 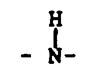 and R¹ is H , and optionally
f) forming a fumarate (and/or a solvate of the fumarate) of compound I, and optionally, and
g) when one or more of R, R¹ and R is a straight or branched alkyl group having 1-5 carbon atoms alkylating the compound obtained in step e) or f).

### Detailed description of the invention

The starting material 4-benzyloxy-3-nitro-styreneoxide (4-benzyloxy-3-nitro-phenyloxirane) (II) is prepared according to the method described in J. Med. Chem. 17, 49 (1974) by C. Kaiser et al. 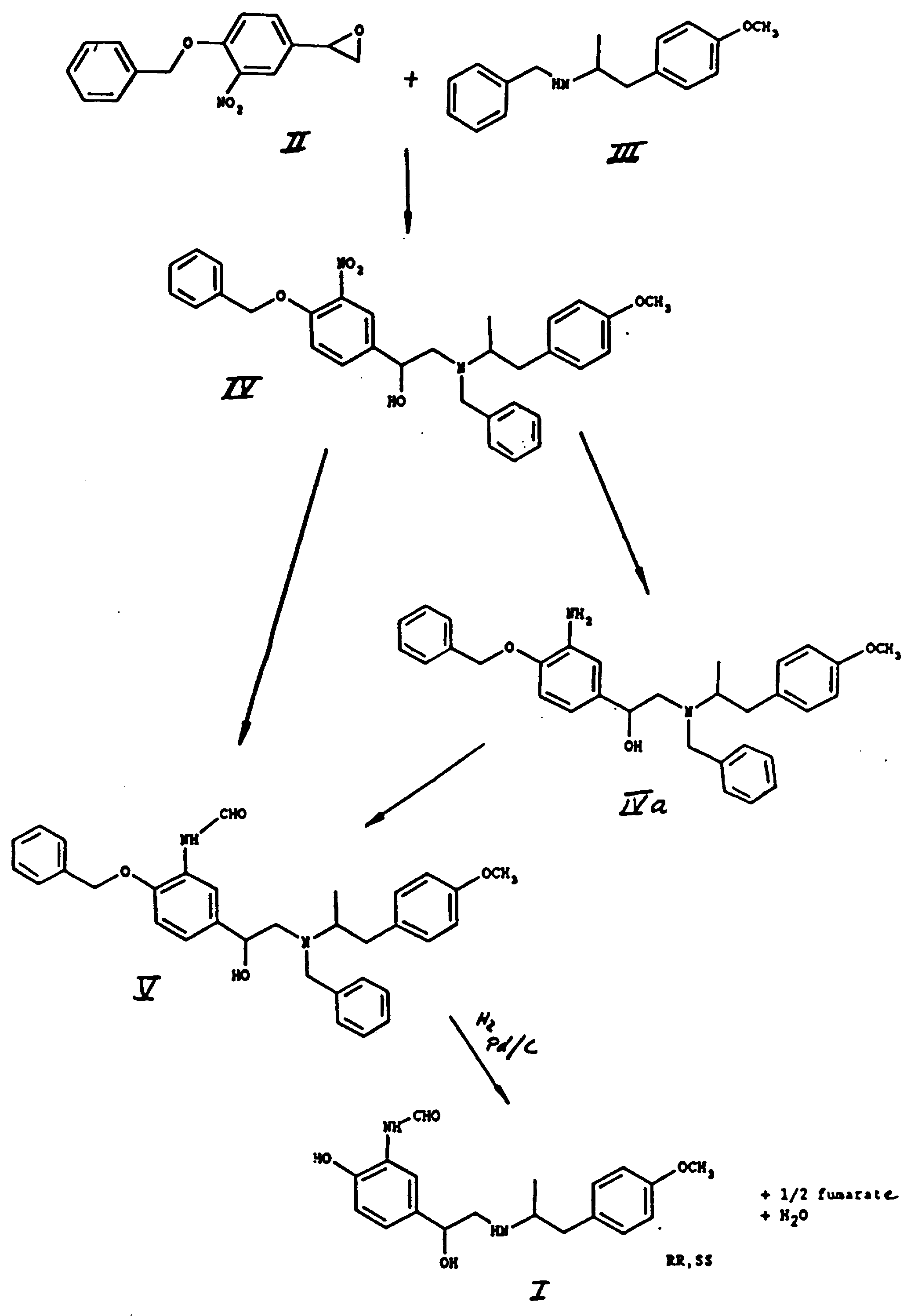 According to the present invention formoterol, i.e. the compound of formula I wherein R and R¹ are hydrogen, is prepared by a) reacting 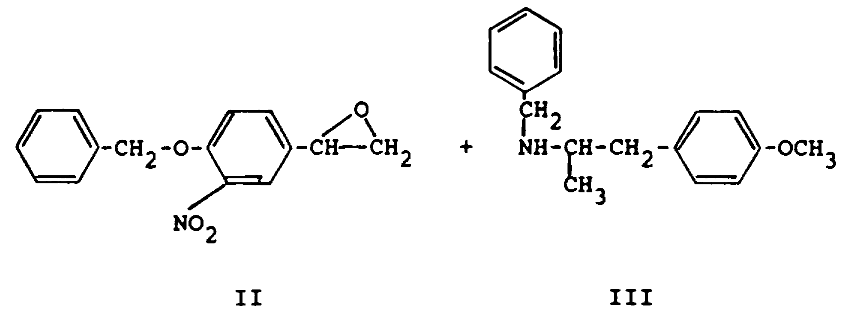 in the presence of a solvent such as a lower alcohol at reflux temperature, or in the absence of a solvent at a temperature of preferably 80-120°C, to give compound IV 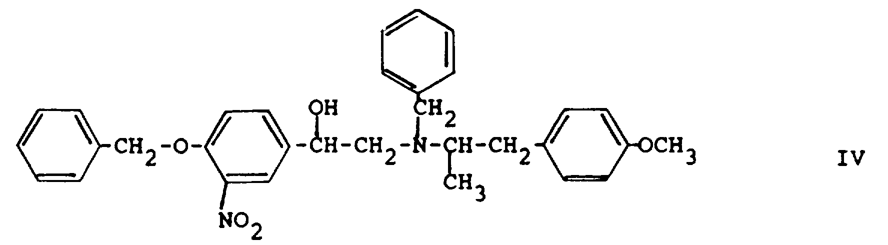
b) reducing and formylating compound IV to give compound V in either a one step reaction with e.g. Raney-nickel/formic acid, or in a two step reaction (catalytic reduction of the nitro group, e.g. over platinum metal in a polar solvent, such as a lower alcohol and/or ester, preferably PtO₂ in methanol or more preferably Pt/C in ethyl acetate, to give compound IVa, followed by formylation, e.g. in a 6-10 fold excess of formic acid). The formylation step is preferably performed at a temperature of 20-90°C during 1-24 hrs. The mixture of the two racemates of V (RR + SS and RS + SR resp.) are thereafter separated by
c) transforming the amine base into its fumarate salt for separation by fractional crystallization in a solvent system comprising ethyl acetate, isopropyl acetate and/or methyl isobutylketone. A very efficient system is based upon a solvent system which also comprises small amounts (preferably less than 15 %) of solvents more polar than the solvents just mentioned, such as dimethylformamide (DMF), dimethylsulfoxide (DMSO) and/or methanol. A preferred solvent system comprises ethyl acetate and a small amount of dimethylformamide, dimethylsulfoxide or methanol. The presence of a solvent like DMF is very desireable in order to increase the solubility and thereby improve the purity of the desired racemate.

Step e) is a common hydrogenolysis of the protecting benzyl groups of the amine base V, performed in a traditional manner with platinum, palladium, or nickel catalysts in a polar solvent, such as ethanol, isopropanol or ethylacetate or a mixture of said solvents, at normal or higher temperature and pressure. A simple crystallization of the formoterol base by means of isopropanol or preferably by means of an isopropanol/water mixture may be carried out in connection with this step as a work up procedure. Said crystallizion should be carried out at a temperature as low as possible (e.g. < 45°C).

The formoterol may thereafter in step f) be transformed into its fumarate salt (and/or a solvate of the fumarate) and crystallized in a polar solvent (isopropanol, ethanol and the like) containing some water (2-25%, preferably 20 %) in order to obtain suitable solid state properties of the compound (hydrate) for the micronization procedure. The compounds usually exist as solvates (hydrates) giving the possibility of different crystal arrangements (polymorphism).

The solvents may or may not be completely evaporated in each step in the process. It is preferred for technical reasons not to evaporate the solvents.

In case one or more of R, R¹ and R in formula I is a straight or branched alkyl group having 1-5 carbon atoms, such as methyl, ethyl, propyl, iso-propyl, a further step g) is necessary, wherein the compound obtained in step e) or step f) is alkylated in a conventional manner, such as by nucleophilic substitution with alkyl halides or by reductive alkylation.

R in the definition of compound I is preferably hydrogen or a straight alkyl group of 1 to 3 carbon atoms.

### Examples

The invention is further illustrated but not limited by the following examples.

### Test methods

The purity of the synthesized compounds has been determined on a HPLC (high performance liquid chromatography) system with UV detection using a LiChrosphere RP Select B 5 um (125 x 4 mm) column under the following conditions:
System A:
   - Mobile phase: : CH₃CN:phosphate buffer pH 4.95 (55:45)
   - Detection wavelength: : 280 nm
   - Flow rate: : 1.0 ml/min
   - Retention time (min): : II 4.8; III 1.9; IV 15 (RR,SS) 17 (RS,SR); IVa 6.7; V 5.5
System B:
   - Mobile phase: : CH₃CN:ethanol:phosphate buffer pH 4.95 (24:16:60)
   - Detection wavelength: : 280 (220) nm
   - Flow rate: : 1.0 ml/min
   - Retention time (min): : V 40 (RR,SS) 48 (RS,SR)
System C:
   - Mobile phase: : CH₃CN:ethanol: 0.1 M NH₄OAc in 0.1 M HOAc (11:11:78)
   - Detection wavelength: : 280 nm
   - Flow rate: : 0.8 ml/min
   - Retention time (min): : I 8.7

The reactions have continously been followed by HPLC and the given data for yield, purity are taken directly from the chromatogram unless otherwise stated.

### Preparation

### Synthesis of IV. (step a)

4-Benzyloxy-3-nitro-styrenoxide (II, 0.5 mole, 135.8 g) and p-methoxyphenyl-2-benzylaminopropane (III, 0.55 mole, 140.5 g) were stirred under nitrogen atmosphere at 90°C for 69 hours (no solvent). The dark syrup (267.0 g) consisted of 94 % of compound IV with a racemic ratio of 43:57 (System A).

### Synthesis of V. (step b)

Method A (two steps). The dark syrup (IV, 133.5 g) in methanol (1.5 1) was hydrogenated over 1.5 g PtO₂ xH₂O at a pressure of 2-4 bar for 3 hours. Compound IVa with a purity of 91 % was obtained (System A).
Filtration and evaporation of the solvent followed by addition of a seven fold excess of formic acid gave after standing for 24 hours at 20°C compound V with a purity of 87 % V (System A) after evaporation. The reaction mixture was used without further purification.

### An alternative of Synthesis of IV and V

4-Benzyloxy-3-nitro-styreneoxide(II, 164 mole, 44.5 kg) and p-methoxyphenyl-2-benzylaminopropane (III, 179 mole, 45.8 kg) were stirred under nitrogen atmosphere at 90°C for 65 hours (no solvent). To the dark syrup dissolved in ethyl acetate (350 1) was added Pt/C (5.6 kg). Hydrogenation with hydrogen gas gave after evaporation of the solvent an oily solid of V with 91.3 % purity. The product was used without further purification.

### Method B (one step)

The dark syrup (IV, 5.0 g), Raney nickel (5 g) and 75 % (v/v) aqueous formic acid (50 ml) were refluxed for 1 hr. The mixture was filtered, and the filtrate and washings diluted with water and extracted with chloroform. The extracts were washed with saturated sodium hydrogen carbonate, water and dried (Na₂SO₄). The oily residue was chromatographed on a silica column using petroleum ether (40-60° C)/ethyl acetate (7.5:6) as eluent giving pure compound V (1.8 g).

### Separation of the racemates (step c)

To fumaric acid (0.62 mole, 72.5 g) dissolved in methanol (2 1) the reaction mixture consisting of compound V (about 1.25 mole) in formic acid was added. After evaporation of the solvents at 50°C the brown syrup was dissolved in ethyl acetate (7.5 1) while heating. The light brown crystals of compound V fumarate formed during cooling were filtered, washed with ethyl acetate (1 1) and dried in vacuum at 40°C for 20 hours. The product (1.12 mole, 654.7 g) had a racemic ratio of 84:16 (System B).
1.10 mole (644.7 g) of compound V fumarate in ethyl acetate (7.5 1) was heated to reflux, DMF (750 ml) was added and the clear solution was allowed to stand for 4 days at room temperature. The light brown crystals formed, were washed with ethyl acetate (1.5 1) and dried in vacuum at 40°C for 4 hours giving a racemic ratio of 98.5:1.5. By repeating the recrystallization procedure, a racemic purity of compound V of almost 100 % was obtained in good yield (System B).

### Synthesis of I.

### Step d

The protected (N,O-dibenzylated) formoterol base (V) was obtained by an extraction procedure (ethyl acetate (2 1)/ammonia, 2 M (1.5 1)) from compound V fumarate (0.28 mole, 164.1 g).

### Step e

Hydrogenolysis of compound V (0.28 mole) in ethanol (2.5 1) with 5 % Pd/C (16.0 g) at 45 psi over night gave the formoterol base (0.23 mole, 78 g (82 %); purity 99.2 %) after filtration and evaporation of the solvent. Recrystallization in isopropanol (1.1 1) increased the purity to 99.8 %. An alternative procedure for recrystallization giving even better purity (>99.9 %) involves the use of a mixture of isopropanol/water (e.g. 85:15) and keeping the temperature as low as possible (e.g. <45°C).

### Step f

To formoterol base (0.176 mole, 60.6 g) in isopropanol (960 ml) and water (200 ml) fumaric acid (0.088 mole, 10.21 g) was added. A clear solution was obtained after heating. After standing at room temperature the crystals were filtered, washed with isopropanol (2 x 50 ml) and dried in vacuum at 45°C for two days. The formoterol fumarate dihydrate formed (0.17 mole, 69.7 g) had a purity of 99.2 % and an diastereomeric purity of 99.8 % (System C). ¹H-NMR (Varian VXR 300, deuterated dimethylsulfoxide, chemical shift in ppm): -CH(C**H**₃)- 0.99 (3H, doublet); - C**H**(CH₃)- 3.0-3.1 (1H, multiplet); -C**H**(OH)- 4.60-4.65 (1H, multiplet); -C**H**₂-NH- 2.75-2.9 (2H, multiplet); -CH(CH₃)-C**H**₂-2.44-2.5, 2.75-2.9 (2H, two multiplets); -OC**H**₃ 3.73 (3H, singlet); -C**H**O 9.61 (1H, doublet); -N**H**-CHO 8.29 (1H, doublet).; -C**H**=C**H**- 6.49 (1H, singlet). In the thermospray mass spectrum the assignment fragments are m/z 345 (MH⁺, 100 %); 327 (MH⁺ - H₂O, 27 %); 166 (C₁OH₁5N₃O⁺, 7.2 %), which confirm the structure.

### Synthesis of N-[2-(3-formamido-4-hydroxy-phenyl)-2-hydroxy-ethyl]-N,N-dimethyl-N-(p-methoxy-α-methyl phenylethyl)ammonium fumarate

A mixture of formoterol fumarate (500 mg), sodium carbonate (200 mg), methyl iodide (5 ml) and acetonitrile (50 ml) was stirred over night at room temperature. Filtration and evaporation of the filtrate gave an oily solid which was washed with chloroform to give 341 mg (66 %) of the crude N-[2-(3-formamido-4-hydroxy-phenyl)-2-hydroxy-ethyl]-N,N-dimethyl-N-(p-methoxy-α-methylphenylethyl)ammonium fumarate. Recrystallization from 95 % ethanol gave a purity of 98.6 % (HPLC system: Licrosphere RP Select B, 5 µm, 129x4 mm, 214 nm, gradient elution from acetonitrile/phosphate buffer (50 mM, pH 3) 16/84 to acetonitrile/phosphate buffer (70/30), 1 ml/ min). ¹H-NMR: -CH(CH₃)- 1.17 (3H, doublet); N-CH₃ 3.2 (3H, singlet); N-CH₃ 3.3 (3H, singlet); -CH(CH₃) 4.0 (1H, multiplet); -CH(OH) 5.1-5.2 (1H, broad doublet); -CH(OH) 6.1 (broad singlet); -OCH₃ 3.76 (3H, singlet); CHO 8.31 (1H, singlet); -NHCHO 9.6 (1H, singlet); -CH=CH- 6.9 (1H, singlet). The electrospray mass spectrum shows the molecular peak at m/z 373 which is in accordance with the proposed structure.

The new process is easier to use and gives an end product with a higher purity than the same compounds prepared according to earlier known processes.

## Claims

1. A process for preparing a compound of the general formula I 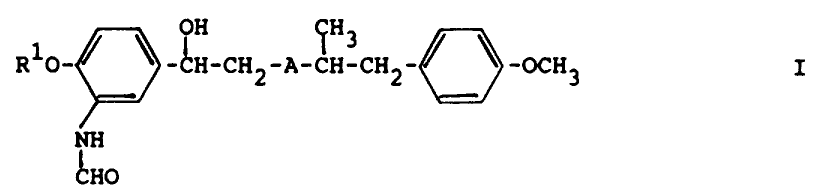
wherein R¹ is H or a straight or branched alkyl group having 1-5 carbon atoms,
A is either 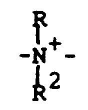 wherein R is H or a straight or branched alkyl group having 1-5 carbon atoms and R is H or a straight or branched alkyl group having 1-5 carbon atoms; or 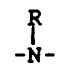 wherein R is as defined above,
or a pharmaceutically acceptable fumarate (and/or a solvate of the fumarate) thereof, in the form of the pure racemates, by
a) reacting 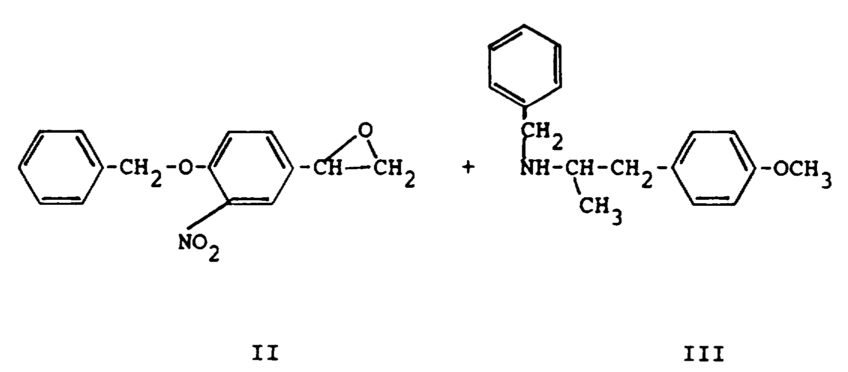 to give compound IV 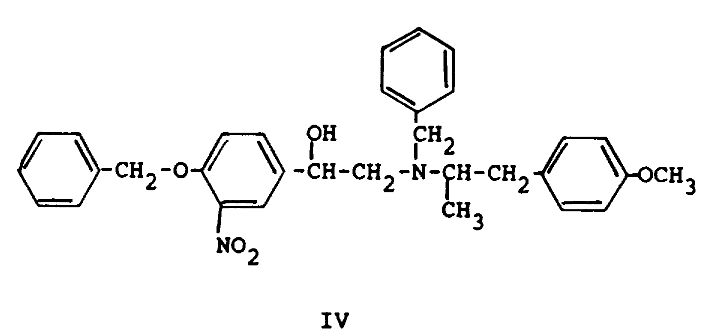
b) reducing and formylating compound IV to give compound V 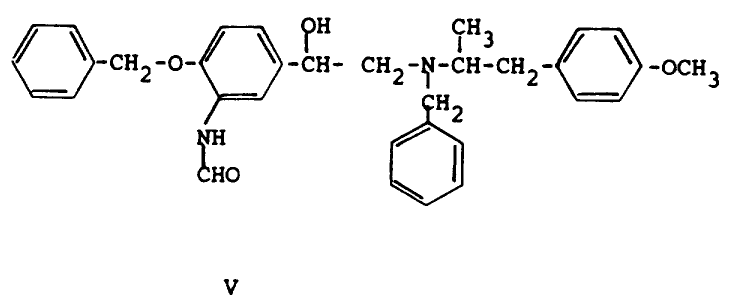
c) forming the fumarate of compound V and resolving the racemates by crystallization,
d) extracting the base of the desired racemate of compound V,
e) hydrogenolyzing of the protecting benzyl groups of said base of compound V to obtain compound I wherein A is 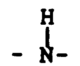 and R¹ is H, and optionally
f) forming a fumarate (and/or a solvate of the fumarate) of compound I, and optionally
g) when one or more of R, R¹, and R is a straight or branched alkyl group having 1-5 carbon atoms alkylating the compound obtained in step e) or f).

2. A process according to claim 1, characterized in that step a) is carried out in the presence of a solvent at reflux temperature or in the absence of a solvent at a temperature of 80-120°C.

3. A process according to either of claims 1-2, characterized in that step b) is a one step reaction with Raney-nickel/formic acid or a two step reaction comprising catalytic reduction of the nitro group followed by formylation in excess of formic acid.

4. A process according to any one of claims 1-3, characterized in that the resolving of the racemates by crystallization in step c) is carried out in a solvent system comprising ethyl acetate, isopropyl acetate and/or methyl isobutylketone.

5. A process according to claim 4, characterized in that the solvent system further comprises small amounts of solvents more polar than the solvents defined in claim 4.

6. A process according to claim 5, characterized in that the more polar solvent is dimethylformamide, dimethylsulfoxide and/or methanol.

7. A process according to claim 5 or claim 6, characterized in that the amount of the more polar solvent is less than 15%.

8. A process according to any one of the preceding claims characterized in that R is hydrogen.

9. A process according to any one of claims 1-7, characterized in that one or more of R, R¹, and R is a straight or branched alkyl group having 1-5 carbon atoms.

## Patentansprüche

1. Verfahren zur Herstellung einer Verbindung der allgemeinen Formel (I) 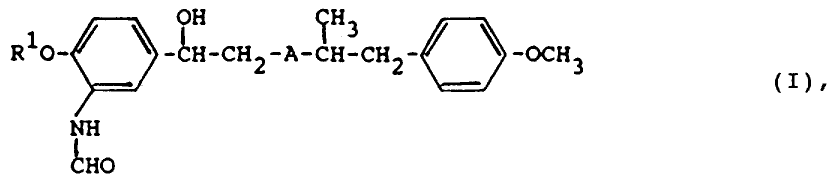
worin R¹ H oder eine gerade oder verzweigte Alkyl-Gruppe mit 1 bis 5 Kohlenstoffatomen bedeutet,
A darstellt: entweder 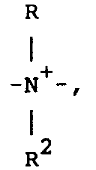 wobei R H oder eine gerade oder verzweigte Alkyl-Gruppe mit 1 bis 5 Kohlenstoffatomen ist, und R H oder eine gerade oder verzweigte Alkyl-Gruppe mit 1 bis 5 Kohlenstoffatomen bedeutet; oder 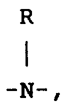 wobei R wie oben definiert ist, oder eines pharmazeutisch annehmbaren Fumarats (und/oder eines Solvats des Fumarats) hievon, in Form der reinen Racemate, durch
a) Umsetzen von 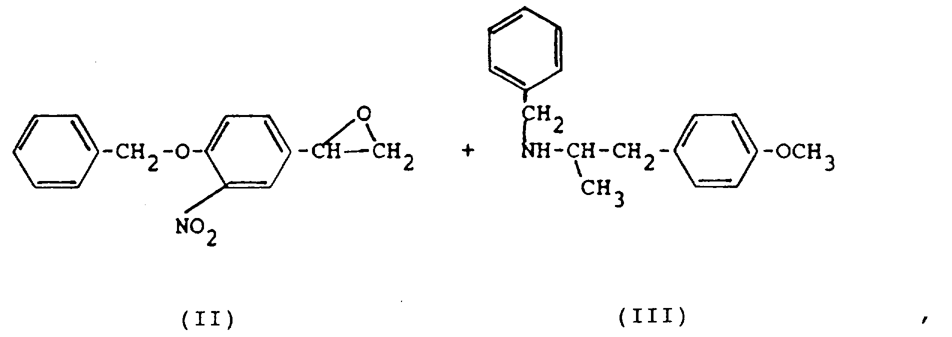 wobei Verbindung (IV) 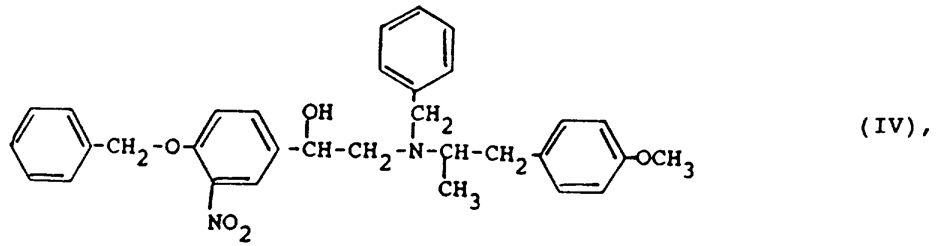 erhalten wird,
b) Reduzieren und Formylieren von Verbindung (IV), wobei Verbindung (V) 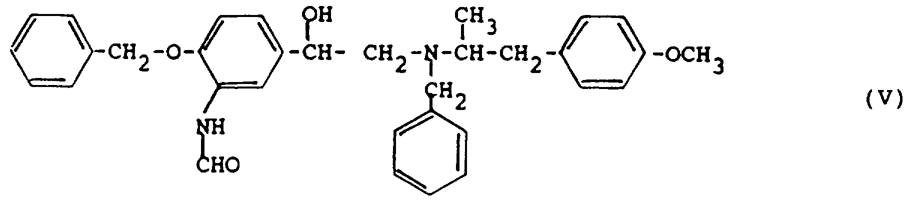 erhalten wird,
c) Bilden des Fumarats von Verbindung (V) und Trennen der Racemate durch Kristallisation,
d) Extrahieren der Base des gewünschten Racemats von Verbindung (V),
e) Hydrogenolysieren der Benzyl-Schutzgruppen der Base von Verbindung (V), wobei Verbindung (I), worin A 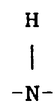 darstellt, und R¹ H ist, erhalten wird, und gegebenenfalls
f) Bilden eines Fumarats (und/oder eines Solvats des Fumarats) von Verbindung (I), und gegebenenfalls,
g) wenn eines oder mehrere von R, R¹ und R eine gerade oder verzweigte Alkyl-Gruppe mit 1 bis 5 Kohlenstoffatomen sind, Alkylieren der in Schritt e) oder f) erhaltenen Verbindung.

2. Verfahren nach Anspruch 1, dadurch gekennzeichnet, daß Schritt a) in Anwesenheit eines Lösungsmittels bei Rückflußtemperatur oder in Abwesenheit eines Lösungsmittels bei einer Temperatur von 80 bis 120°C durchgeführt wird.

3. Verfahren nach einem der Ansprüche 1 und 2, dadurch gekennzeichnet, daß Schritt b) eine einstufige Reaktion mit Raney-Nickel/Ameisensäure oder eine zweistufige Reaktion, umfassend die katalytische Reduktion der Nitro-Gruppe, gefolgt von Formylieren in einem Ameisensäure-Überschuß, ist.

4. Verfahren nach einem der Ansprüche 1 bis 3, dadurch gekennzeichnet, daß die Trennung der Racemate durch Kristallisation in Schritt c) in einem Lösungsmittelsystem, umfassend Ethylacetat, Isopropylacetat und/oder Methylisobutylketon, durchgeführt wird.

5. Verfahren nach Anspruch 4, dadurch gekennzeichnet, daß das Lösungsmittelsystem ferner geringe Mengen an Lösungsmitteln, die polarer sind als die in Anspruch 4 definierten Lösungsmittel, umfaßt.

6. Verfahren nach Anspruch 5, dadurch gekennzeichnet, daß das polarere Lösungsmittel Dimethylformamid, Dimethylsulfoxid und/ oder Methanol ist.

7. Verfahren nach Anspruch 5 oder 6, dadurch gekennzeichnet, daß die Menge des polareren Lösungsmittels geringer ist als 15 %.

8. Verfahren nach einem der vorhergehenden Ansprüche, dadurch gekennzeichnet, daß R Wasserstoff bedeutet.

9. Verfahren nach einem der Ansprüche 1 bis 7, dadurch gekennzeichnet, daß eines oder mehrere von R, R¹ und R eine gerade oder verzweigte Alkylkette mit 1 bis 5 Kohlenstoffatomen darstellt.

## Revendications

1. Procédé de préparation d'un composé de formule générale I: 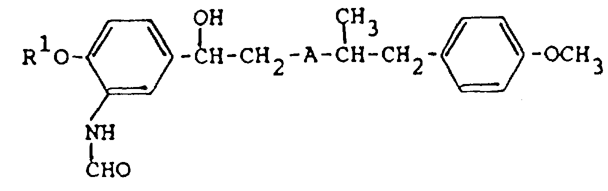
dans laquelle R¹ est H ou un groupe alkyle linéaire ou ramifié comportant 1-5 atomes de carbone,
A est 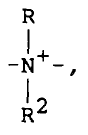 où R est H ou un groupe alkyle linéaire ou ramifié comportant 1-5 atomes de carbone et R est H ou un groupe alkyle linéaire ou ramifié comportant 1-5 atomes de carbone; ou bien 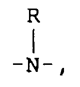 où R est tel que défini ci-dessus,
ou d'un de ses fumarates (et/ou d'un des solvates du fumarate), pharmaceutiquement acceptable, sous forme de racémates purs, qui consiste à:
a) faire réagir 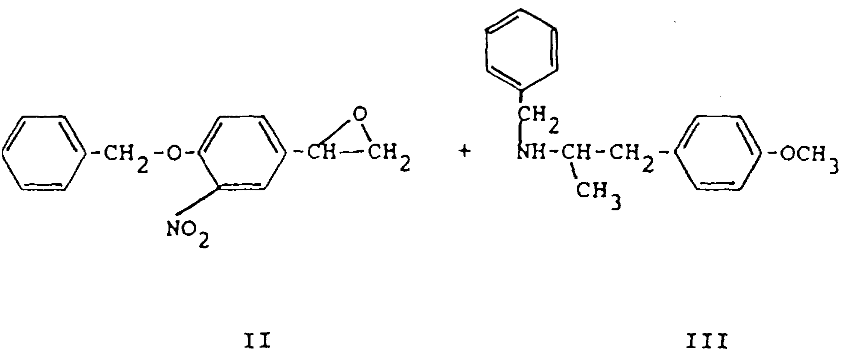 pour obtenir le composé IV: 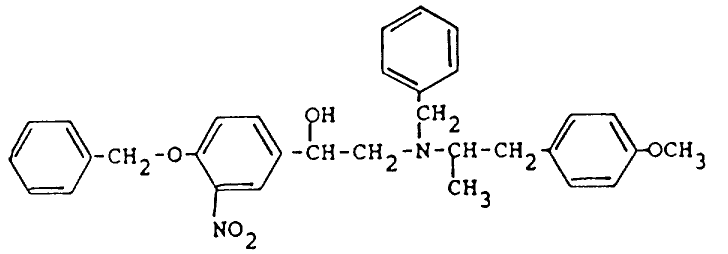
b) réduire et formyler le composé IV pour obtenir le composé V: 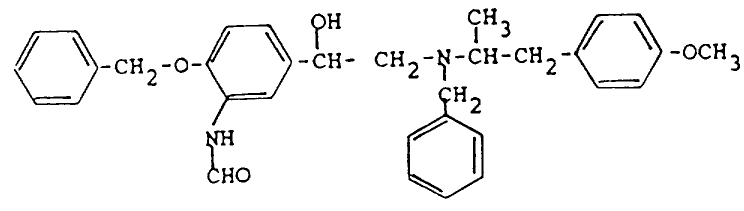
c) former le fumarate du composé V et résoudre les racémates par cristallisation,
d) extraire la base du racémate recherché du composé V,
e) hydrogénolyser les groupes benzyle protecteurs de ladite base du composé V pour obtenir un composé I, dans lequel A est 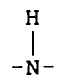 et R¹ est H, et, le cas échéant,
f) former un fumarate (et/ou un solvate du fumarate) au composé I, et, le cas échéant,
g) lorsqu'un ou plusieurs des radicaux R, R¹ et R est un groupe alkyle linéaire ou ramifié comportant 1-5 atomes de carbone, alkyler le composé obtenu dans l'étape e) ou f).

2. Procédé selon la revendication 1, caractérisé en ce que l'étape a) est réalisée en présence d'un solvant à la température de reflux ou en l'absence d'un solvant à une température de 80-120°C.

3. Procédé selon l'une quelconque des revendications 1-2, caractérisé en ce que l'étape b) est une réaction en une seule étape avec un système nickel de Raney/acide formique ou une réaction en deux étapes comprenant la réduction catalytique du groupe nitro, suivie d'une formylation dans un excès d'acide formique.

4. Procédé selon l'une quelconque des revendications 1-3, caractérisé en ce que la résolution des racémates par cristallisation dans l'étape c) est réalisée dans un système de solvant comprenant de l'acétate d'éthyle, de l'acétate d'isopropyle et/ou de la méthylisobutylcétone.

5. Procédé selon la revendication 4, caractérisé en ce que le système de solvant comprend aussi de petites quantités de solvants plus polaires que les solvants définis dans la revendication 4.

6. Procédé selon la revendication 5, caractérisé en ce que le solvant plus polaire est le diméthylformamide, le diméthylsulfoxyde et/ou le méthanol.

7. Procédé selon la revendication 5 ou la revendication 6, caractérisé en ce que la quantité du solvant plus polaire est inférieure à 15%.

8. Procédé selon l'une quelconque des revendications précédentes, caractérisé en ce que R est un hydrogène.

9. Procédé selon l'une quelconque des revendications 1-7, caractérisé en ce qu'un ou plusieurs des radicaux R, R¹ et R est un groupe alkyle linéaire ou ramifié comportant 1-5 atomes de carbone.
